# EUROPEAN PATENT SPECIFICATION

(11) **EP 0 672 120 B1**
(45) Date of publication and mention of the grant of the patent: **17.10.2001**
(21) Application number: 93900518.7
(22) Date of filing: 06.11.1992
(51) Int. Cl.: C12N 15/13, C12N 5/20, C12N 5/28, C07K 16/28, C12P 21/08, A61K 39/395, A61K 39/42

(54) **PRODUCTION OF HUMAN MONOCLONAL ANTIBODIES ACTIVE AGAINST HEPATITIS B SURFACE ANTIGEN**
DIE HERSTELLUNG VON MENSCHLICHEN, MONOKLONALEN ANTIKÖRPERN, DIE GEGEN DAS OBERFLÄCHENANTIGEN VON HEPATITIS B AKTIV SIND
PRODUCTION D'ANTICORPS MONOCLONAUX HUMAINS ACTIFS CONTRE L'ANTIGENE DE SURFACE DE L'HEPATITE B

(43) Date of publication of application: 20.09.1995
(73) Proprietor: SANDOZ LTD., 4002 Basel (CH)
(72) Inventor: OSTBERG, Lars, G., Convent Station, NJ 07961 (US)
(74) Representative: Bizley, Richard Edward
(86) International application number: US9209749
(87) International publication number: WO9411495

(56) References cited:
- GB-A- 2 113 715
- US-A- 4 346 073
- HUMAN ANTIBODIES AND HYBRIDOMAS, vol. 3, no. 1, January 1992, pages 2-7, XP000573890 EHRLICH ET AL: "CHARACTERIZATION OF HUMAN MONOCLONAL ANTIBODIES DIRECTED AGAINST HEPATITIS B SURFACE ANTIGEN"
- Gastroenterology, Volume 80, Number 2, issued February 1981, J.R. WANDS et al., "High Affinity Monoclonal Antibodies to Hepatitis B Surface Antigen (HBsAg) Produced by Somatic Cell Hybrids", pages 225 to 232, see entire document.
- The Journal of Immunology, Volume 139, Number 10, issued 15 November 1987, R.F. TIEBOUT et al., "A Human Hybrid Hybridoma", pages 3402 to 3405, see entire document.
- Molecular Immunology, Volume 27, Number 3, issued March 1990, Y. LI et al., "Construction, Expression and Characterization of a Murine/Human Chimeric Antibody with Specificity for Hepatitis B Surface Antigen", pages 303 to 311, see entire document.
- Proceedings of the National Academy of Sciences (USA), Volume 89, Number 8, issued 15 April 1992, S.L. ZEBEDEE et al., "Human Combinatorial Antibody Libraries to Hepatitis B Surface Antigen".
- Clinical Chemistry, Volume 34, Number 9, issued September 1988, P.H. EHRLICH et al., "Human and Primate Monoclonal Antibodies for In Vivo Therapy", pages 1681 to 1688, see entire document.
- Biotechnology, Volume 7, issued April 1989, K. HARADA et al., "Human-Human Hybridomas Secreting Hepatitis B Virus-Neutralizing Antibodies", pages 374 to 377, see entire document.
- Molecular Immunology, Volume 23, Number 9, issued September 1986, A.R. NEURATH et al., "Characterization of Monoclonal Antibodies Specific for the pre-s2 Region of the Hepatitis B Virus Envelope Protein", pages 991 to 997, see entire document.
- The Journal of Immunology, Volume 128, Number 1, issued January 1982, M. IMAI et al., "Demonstration of Two Distinct Antigenic Determinants on Hepatitis B e Antigen by Monoclonal Antibodies", pages 69 - 72, see entire document.
- Biochemical and Biophysical Research Communications, Volume 142, Number 3, issued 13 February 1987, Y. ICHIMORI et al., "Establishment of Hybridoma Secreting Human Monoclonal Antibody Against Hepatitis B Virus Surface Antigen", pages 805 to 812, see entire document.
- Biochemical and Biophysical Research Communications, Volume 129, Number 1, issued 31 May 1985, Y. ICHIMORI et al., "Establishment of Hybridomas Secreting Human Monoclonal Antibodies Against Tetanus Toxin and Hepatitis B Virus Surface Antigen", pages 26 to 33, see entire document.
- J.W. GODING, "Monoclonal Antibodies", published 1983 by Academic Press (London), pages 118 to 125, see entire document.
- Cancer Research, Volume 45, Number 7, issued July 1985, G.L. BURAGGI et al., "Imaging with 131I-Labelled Monoclonal Antibodies to a High-Molecular-Weight Melanoma-Associated Antigen in Patients with Melanoma: Efficacy of Whole Immunoglobulin and its F(ab')2 Fragments", pages 3378 to 3387, see entire document.

## Description

The present invention concerns hybridoma cell lines which produce human antibodies which neutralize the hepatitis B virus, methods for producing the cell lines, antibodies produced by the cell lines, and uses of the antibodies, particularly therapeutically.

The making of hybridoma cell lines for the purpose of producing monoclonal antibodies is in general well known at this time to researchers in this art. The present invention concerns the obtaining of human monoclonal antibodies effective in particular against hepatitis B surface antigen (HBsAg), such antibodies being prepared according to a generally applicable method described by the applicant in Hybridoma 2(4):361 (1983) and United Kingdom Patent Application 2,113,715A, published August 10, 1983. More particularly, it has been found that a hybridoma cell line comprising a parent rodent immortalizing cell, such as a murine myeloma cell, e.g. SP-2, fused to a human partner cell results in an immortalizing xenogeneic hybridoma cell. This xenogeneic hybridoma cell may be fused to a cell capable of producing an anti-HBsAg human antibody, resulting in a novel trioma cell line capable of generating human antibody effective against such antigen in the human. Alternately, when greater stability is desired, a trioma cell line which preferably no longer has the capability of producing its own antibody is made and this trioma is then fused with a further cell capable of producing useful against said antigen so as to obtain a still more stable hybridoma (quadroma) which produces antibody against the antigen.

The applicant's publications earlier referred to describe the preparation of a xenogeneic hybridoma referred to as SPAZ 4, prepared from drug resistant cell line SP-2 obtainable, e.g., from the NIGMS Human Genetic Mutant Cell. Repository Ref. CM35669A (see U.S. DHHS 1982 Catalog of Cell Lines). Preparation of SPAZ 4 is summarized as follows. The SP-2 cell line is fused with normal human peripheral lymphocytes by conventional techniques. A large number of hybrids is obtained and, after approximately five weeks, five clones are selected which show fast growth and no antibody production. These cells are selected for resistance to 8-azaguianine and with three of these lines it is possible to obtain mutants which are resistant to 20 µg/ml of 8-azaguanine. These cells are at the same time sensitive to Hypoxanthine-Aminopterin-Thymidine (HAT) medium which showed that they had lost their ability to produce hypoxanthine phosphoribosyl transferase. One of these cell lines is SPAZ 4.

Cell line SPAZ 4 may be fused with cells obtained from the blood of persons immunized with hepatitis B vaccine to obtain hybridoma cell lines which provide positive cultures when standard selection procedures are used involving binding of antibodies to relevant viral antigens. It is preferred that said positive cultures be placed through a second selection process in which different subtypes of the virus are used for antigen preparation. This provides an opportunity to pinpoint the exact antigenic determinant recognized by the antibody.

The cell lines resulting from the fusion of a xenogeneic hybridoma and the human monoclonal antibody producing cell (trioma) are therefore useful in providing monoclonal antibodies capable of effective activity in neutralizing a virus causing hepatitis, and said antibodies can therefore prevent the spread of hepatitis through e.g. blood transfusion. They can also be used to give initial protection to newborn babies or exposed individuals earlier than a vaccine could be effective. Anti-hepatitis antibodies may be used to protect immunosuppressed patients, including transplantation patients, from recurrent hepatitis. This is most significant in cases of hepatitis B positive liver recipients. Further, the antibodies can be used in diagnostic assays.

Thus, in one aspect the invention comprises a human monoclonal antibody or Fab fragment thereof, which neutralises hepatitis B virus, and has a V_{H} region with the amino acid sequence shown in Table 8-1 and which has a V_{L} region with the amino acid sequence shown in Table 9-1.

In another aspect the invention comprises a human monoclonal antibody that neutralises hepatitis B virus and which has a V_{H} region which has at least 80% sequence identity to that shown in Table 8-1 and which has a V_{L} region which has at least 80% sequence identity to that shown in Table 9-1, wherein the monoclonal antibody inhibits binding of an antibody of claim 1 to hepatitis B surface antigen.

The invention also comprises a hybridoma cell line comprising a xenogeneic immortalising cell fused to a human cell capable of producing an antibody of the invention.

The invention further comprises a method of making a hybridoma cell line of the invention, comprising making a xenogeneic immortalising cell line drug resistant, fusing the resulting drug resistant immortalising cell to a cell capable of producing human antibody, and selecting the desired hybrid.

In a further aspect the invention comprises a cell line which produces an antibody of the invention.

The invention further comprises a method of producing an antibody of the invention comprising obtaining said antibody from a cultured hybridoma cell line of the invention .

In another aspect the invention comprises isolated DNA, optionally as a component of a vector, comprising a DNA sequence which hybridises under stringent conditions to a strand of DNA selected from:
a) a strand of Table 8-1; and
b) a strand of Table 9-1;
wherein the DNA encodes an antibody chain, which as a component of an antibody inhibits binding of an antibody of the invention to hepatitis B surface antigen.

The invention also includes the use of such DNA as a probe or for use in cloning antibodies, and a cell line comprising such DNA.

It has also been found that antibody fragments, such as Fab fragments can also bind to hepatitis B virus surface antigen. These fragments also make up part of this invention.

Thus, in a further aspect of the invention a pharmaceutical composition comprising an antibody or Fab fragment of the invention is provided.

The invention also comprises an antibody or Fab fragment of the invention for use as a pharmaceutical and the use of an antibody or Fab fragment of the invention in the manufacture of a medicament for combating hepatitis B virus and/or reducing the level of circulating hepatitis B surface antigen.

In another aspect, the invention comprises the use of an antibody or Fab fragment of the invention in an in vitro diagnostic assay.

Specific antibodies which have been made include PE1-1, ZM1-1, ZM1-2, MD3-4 and LO3-3, each of these antibodies being of the IgG₁ class.

The cell line producing PE1-1 was deposited at the American Type Culture Collection on October 16, 1986 and given accession number ATCC HB 9234; the cell line producing ZM1-1 was deposited as ATCC HB 9191 on September 4, 1986 and the cell line producing ZM1-2 was deposited as ATCC HB 9192. The address of the American Type Culture Collection is 12301 Parklawn Drive, Rockville, Maryland 20852.

The cell lines of the present invention all behave as typical (mouse x human) x human hybridomas and produce their respective antibodies in concentrations ranging up to 25 mg/l in standard suspension culture.

### DESCRIPTION OF THE FIGURES

Figure 1 shows the results of a direct binding enzyme linked immunoassay comparing binding kinetics of antibody PE1-1 (shown by the single line) and antibody ZM1-2 (double line). Details are given in Example 4A.

Figure 2 shows the serum levels of antibody PE1-1 in rhesus monkey serum determined at various times post-dosage. Details are given in Example 4C.

Throughout the specification and claims, the same designation is given to both the cell line and the antibody it produces, i.e. cell line PE1-1 produces monoclonal antibody PE1-1; cell line ZM1-1 produces monoclonal antibody ZM1-1, etc. It is felt that one of ordinary skill in the art will understand whether the cell line or the antibody is being discussed.

Monoclonal antibody and cell line PE1-1 has also been referred to by the inventor and the inventor's assignee as OST 577 and 64-577. Likewise, monoclonal antibody and cell line ZM1-2 have also been referred to as 265-695, and monoclonal antibody and cell line L03-3 have been referred to as 266-215.

The antibodies and antibody fragments obtained according to this invention have good specificity for hepatitis B surface antigen in in vitro ELISA binding assays.

As the antibodies of the present invention are of human origin, they are advantageously used in human therapy, as no allergenic response develops with repeated therapy, as occurs with murine or ovine antibodies. Thus, another aspect of this invention is a method of treating hepatitis B through the administration of one or more of the aforementioned antibodies. It has been found that repeated doses of approximately 10-40 mg antibody will substantially reduce the amount of circulating HBsAg. Additional doses were found to decrease the amount of HBsAg levels to below the detectable limits of antigen tests.

Another aspect of this invention is a cocktail of two or more monoclonal antibodies. This mixture is particularly suited for administration to patients who carry a non-wild type strain of hepatitis B virus which does not bind well with a given single monoclonal antibody. For example, one compassionate need patient who suffered from hepatocellular carcinoma and chronic hepatitis B was given antibody PE1-1 prior to liver transplantation, and repeated doses thereafter. (Details are given below in Example 5). After approximately four and one-half months of treatment, low levels of serum HBsAg could be detected with a polyclonal antibody, but not with PE1-1. Polymerase chain reaction (PCR) DNA analysis of the 230 base pair region of the HBsAg gene corresponding to the putative monoclonal antibody binding domain was performed. The PCR DNA was cloned into M13 bacteriophage and the resulting DNA was sequenced. Analysis of clones from each of the serum samples revealed two variant sequences when compared to PCR DNA from the original liver and pre-antibody therapy. The variant DNA codes for two different amino acids in the S protein of HBsAg and also codes for a stop codon (UAG) in the viral polymerase gene. Both variant genes contain an amino acid change resulting in the substitution of arginine for glycine in a conserved peptide domain.

Since the monoclonal antibodies PE1-1, ZM1-2, ZM1-1, MD3-4 and L03-3 have been shown to bind to different epitopes, and at least one of the monoclonal antibodies has been found to bind to every variant virus tested to date to a sufficient extent to render it clinically useful, another aspect of this invention is a cocktail of two or more of the monoclonal antibodies selected from the group consisting of: PE1-1, ZM1-2, ZM1-1, MD3-4 and L03-3. Particularly preferred are cocktails of two monoclonal antibodies, especially the mixture of PE1-1 and ZM1-2 and the mixture of PE1-1 and L03-3. The ratio of the monoclonal antibodies present in the mixture may vary depending on many factors apparent to one of ordinary skill in the art and include: the genotype of the hepatitis virus or viruses present in the patient's serum, the relative binding strengths of the antibodies chosen, the epitopes to which the chosen antibodies bind, and economic considerations. Generally, the antibodies will be present in a ratio ranging from 1:99, more typically from 25:75, and preferably in a substantially equal amount.

Sections of the PE1-1, ZM1-1, ZM1-2 and MD3-4 were sequenced using standard techniques. The sequence obtained for the V_{H} region of PE1-1 is given in Table 8-1, and areas corresponding to the CDR1, CDR2, and CDR3 (D_{H} and J_{H4}) are noted. As the CDR regions are particularly important regions in determining the binding properties of an antibody, this invention includes an antibody that has an amino acid sequence of its CDR1 region which is substantially similar to that of PE1-1, as set forth in Table 8-1. This invention also includes an antibody that has an amino acid sequence in its CDR2 region which is substantially similar to that of PE1-1, as set forth in Table 8-1. Further, this invention also comprises an antibody that has an amino acid sequence of its CDR3 region which is substantially similar to the CDR3 region of PE1-1, as set forth in Table 8-1.

Likewise, the V_{H} region of ZM1-1 was sequenced as is given in Table 8-2. Areas corresponding to its CDR1, CDR2, and CDR3 (D_{H} and J_{H4}) are also indicated. This invention includes an antibody which has an amino acid sequence of its CDR1 region which is substantially similar to that of ZM1-1 as set forth in Table 8-2. Also, this invention includes an antibody which has an amino acid sequence of its CDR2 region which is substantially similar to that of ZM1-1 as set forth in Table 8-2, and further this invention also comprises an antibody that has an amino acid sequence of its CDR3 region which is substantially similar to that of ZM1-1 as set forth in Table 8-2.

The DNA sequences which code for the regions of ZM1-2 and MD3-4 are given in Table 8-3 and 8-4 respectively. This invention also includes any antibody which has amino acid sequences which are substantially similar to that of the regions of ZM1-2 and MD3-4 as set forth in Tables 8-3 and 8-4.

The DNA sequences which code for the V_{H} regions of PE1-1, ZM1-1, ZM1-2 and MD3-4 were determined and appear in Tables 8-1 and 8-2, 8-3 and 8-4 respectively. These sequences or appropriate fragments may be used in cloning antibodies (or modified antibodies) or as probes. Antibodies which are produced through genetic engineering processes (rather than conventional harvesting from hybridomas) can be made using cloning techniques which are known in the art. DNA from other sources may be used to produce a synthetic antibody molecule which retains the binding characteristics of PE1-1, ZM1-1, ZM1-2 and MD3-4 by virtue of having substantially similar CDR1, CDR2, and/or CDR3 regions. Such antibodies are within the scope of this invention.

The DNA sequences which code for the v_{L} light chain variable regions of PE1-1, ZM1-1, ZM1-2 and MD3-4 are given in Tables 9-1, 9-2, 9-3, end 9-4, respectively. This invention also includes any antibody which has amino acid sequences which are substantially similar to that of the regions of PE1-1, ZM1-1, ZM1-2 and MD3-4 as set forth in Tables 9-1, 9-2, 9-3 and 9-4.

Also within the scope of this invention are the DNA sequences which code for the v_{H} region, the v_{L} region, the CDR1 regions, the CDR2 regions and/or the CDR3 regions of PE1-1, 2M1-1, ZM1-2 and MD3-4. Also included is DNA which would hybridize to any of the aforementioned sequences under stringent hybridization conditions. This DNA is substantially free from other DNA of the donor mammal, and may contain introns or it may be cDNA.

As used throughout the specification and claims, the following definitions are intended. An amino acid sequence is "substantially similar" to another amino acid sequence if their amino acid homology is at least 80%. Referring to DNA, "stringent hybridization conditions" are those in which hybridization is effected at 60°C in 2.5 X saline citrate buffer (SSC) followed merely by rinsing at 37°C at a reduced buffer concentration which will not affect the hybridizations which take place. "Associated mammalian DNA" means DNA present in the mammal which is the source of the v_{H} antibody chain, but which is not involved in coding for an antibody or antibody fragment.

The invention is more fully exemplified in the following non-limiting examples.

### EXAMPLE 1

### PRODUCTION OF ANTIBODY CELL LINES

Human volunteers are immunized with hepatitis B vaccine. MD3-4, ZM1-2, ZM1-1, and PE1-1 hybridoma cell lines are derived from lymphocytes of individuals immunized with Heptavax® (Merck & Co.). Cell line L03-3 is developed from cells of an individual injected several times with Heptavax®, and just preceding the fusion, Recombivax® (Merck & Co.). Peripheral blood lymphocytes are purified by density gradient centrifugation on a cushion of Percoll (Pharmacia Inc.), density 1.085 g/ml. The isolated lymphocytes are washed three times in Hank's Balanced Salt Solution and mixed with an equal number of cells from (mouse x human) cell line SPAZ-4. The cell mixture is pelleted at room temperature with 400 x g for 5 minutes. After removing the medium, the cell pellet is treated with a 50% solution of PEG-1000 in Dulbecco's Minimal Essential Medium (MEM) for 1 minute at 37°C after which the medium was slowly diluted with Dulbecco's MEM. The cells are collected by centrifugation and resuspended into Dulbecco's MEM containing 20% fetal bovine serum. The cells are seeded at approximately 2 X 10⁶ cells per ml into microwell plates. On the following day fresh medium containing the components of HAT medium (hypoxanthine aminopterin thymidine) is added in order to select against non-fused SPAZ-4 cells. On day 4 after fusion the medium is replaced with fresh medium containing only HT as all cells sensitive to HAT-selection had been killed by that time.

After 3 to 4 weeks, when guod growth of hybridoma-like cells could be seen microscopically, supernatants are tested for the presence of anti-hepatitis B surface antigen antibody. An ELISA-assay using a 1/100 dilution of Heptavax® on the solid phase is used. After incubation with the supernatants the plates are developed with a kit of biotinylated goat anti-human immunoglubulin and avidin-coupled horseradish peroxidase (Vectastain®, Vector Laboratories'Inc.). The enzyme is detected by the color reaction with phenylenediamine. Positive cultures are picked into new wells and a part of the cells is cloned by limiting dilution in Dulbecco's MEM containing 20% fetal bovine serum and 10⁷ mouse thymocytes per milliliter. The cloning plates are tested by the same ELISA method as described above and positive cultures are expanded and frozen.

All the cell lines behave as typical (mouse x human) x human hybridomas and produce their respective antibodies in concentrations ranging up to 25 mg/l in standard suspension culture.

### EXAMPLE 2

### IMMUNOCHEMICAL CHARACTERIZATION

### A. Antibody Class/Subclass

The immunoglobulin class of antibodies PE1-1, ZM1-1, ZM1-2, MD3-4 and L03-3 is determined using ELISA methodology. Each antibody is captured on an antigen-coated plate and each assay is developed with subclass specific, peroxidase-conjugated anti-human Ig (Tago). Each of the antibodies are clearly IgG₁.

### B. Light Chain Type

Using ELISA methods similar to those described in A, above, each antibody is tested with anti-κ or anti-λ light chain reagents (Tago). The following results are obtained.

| | |
|---|---|
| PE1-1 | lambda |
| ZM1-1 | kappa |
| 2M1-2 | kappa |
| L03-3 | lambda |
| MD3-4 | lambda |

### C. Isoelectric Focusing (IEF)

A sample of antibody L03-3 or PE1-1 is applied to gel. Each is found to behave as a basic protein.

### D. Specificity

purified HBsAg of subtypes adw and ayr are purchased from Scripps Laboratories, San Diego, California. HBsAg subtype ayw is obtained from Connaught Laboratories (Willowdale, Ontario). ELISA assays are performed essentially as described by Ostberg, et al. (1983) Hybridoma 2:361-367.

PE1-1 reacts with both ayr and adw, but it reacts slightly better with the adw subtype. L03-3 reacts substantially equally well with ayr and adw. ZM1-1 shows higher reactivity with adw, but ZM1-2 binds slightly better to ayr. These results are confirmed for PE1-1 and L03-3 by Scatchard analysis in solid phase RIA with solid adsorbed ayr or adw antigen. Thus, although these monoclonal antibodies apparently do not bind to the subtypic determinant, their reaction with HBsAg can be significantly affected by the subtype.

### G. Allotype Determination

Allotypes are determined using reagents supplied by the Central Laboratory of the Netherlands Red Cross Transfusion Service. Inhibition ELISA or direct binding ELISA are used. Results are presented in Table 1, below. As can be seen, there is no apparent restriction on high affinity anti-HBsAg antibodies with respect to light chain or allotype.

**TABLE 1**

| Allotypes of Anti-HBsAg Monoclonal Antibodies | | | | |
|---|---|---|---|---|
| | Allotypes | | | |
| Antibody | a | f | z | Km(3) |
| PE1-1 | - | + | - | * |
| ZM1-2 | + | - | + | + |
| L03-3 | - | + | - | * |
| ZM1-1 | ND | ND | ND | + |
| ND = Not determined | | | | |
| * = Antibody has λ light chain which does not have Km allotypes | | | | |

### G. Affinity

The affinity for solid adsorbed HBsAg is determined for each antibody using radiolabelled antibodies essentially as described by Wands, et al. (1981) Gastroenterology 80:225-232, which is hereby incorporated by reference. Antibodies are labeled with ¹²⁵I with Iodogen (Pierce). For each monoclonal except L03-3, the solid phase absorbed HBsAg is ayw. L03-3 is assayed with both ayr and adw with essentially the same results. Antibody-antigen incubation occurs at room temperature.

The relative affinity is also determined using an inhibition ELISA in which varying concentrations of soluble HBsAg (ayw subtype) are pre-incubated with monoclonal antibody and the mixture is then incubated at 37°C in a microtiter well coated with HBsAg. Results are presented below in Table 2.

**TABLE 2**

| Affinity of Monoclonal Antibodies for HBsAg | | |
|---|---|---|
| Antibody | Solid Phase RIA, M⁻¹ | Inhibition ELISA, M⁻¹ |
| PE1-1 | 3.6 X 10⁹ | ~2 X 10⁹ |
| ZM1-2 | 1.5 X 10⁹ | ~7 X 10⁸ |
| L03-3 | 1.7 X 10⁹ | ~1 X 10⁸ |
| ZM1-1 | 5 X 10⁹ | ~1 X 10⁸ |

As can be seen from the table above, for both PE1-1 and ZM1-2 the ELISA results are approximately two-fold lower than the RIA results, which is within the range of experimental error. A Scatchard plot of the results of the RIA performed on ZH1-1 indicates that there might be a low affinity binding site. It is thus possible that the ELISA is measuring this low affinity binding site, as the ELISA results are some 50-fold lower than the RIA. In addition, Scatchard plots also indicate that there are considerably less high affinity ZM1-1 sites than ZM1-2 or PE1-1 high affinity sites. While not wishing to be bound by theory, it appears that ZM1-1 may have the highest affinity for HBsAg of the four antibodies compared, but only for HBsAg in a certain spatial arrangement. This arrangement is only manifested in a small percentage of HBsAg molecules. It is also possible that this may be due to bivalent binding of ZM1-1 to HBsAg while the low affinity site is monovalent.

### EXAMPLE 3

### Potency of PE1-1

Antibody PE1-1 is tested for potency in the AUSAB radioimmunoassay (Abbott). Tests are performed against the Bureau of Biologics Reference Hepatitis B immune globin, and several commercial hepatitis B immune globulin preparations (H-Big Immune Globin®, Hep B Gammagee Immune Globin®, and Hyper Hep Immune Globin®, all purchased from a pharmaceutical supply house). Despite the fact that the immune globulin preparations are polyclonal and PE1-1 is monocional, the binding data are within the criteria of the Bureau of Biologics for comparing immune globulin preparations, i.e., the lines were parallel at a probability level less than or equal to 0.01.

Determination of potency is as follows. Preparations are compared on a weight basis (an absorbance at 208 nm of 1.4 is assumed equal to 1 mg/ml). Preparations of PE1-1 which have been stored at 5°C are then compared with the above polyclonal preparations which have also been stored at 5°C. The logarithm of 1000 divided by µg/ml in the preparation (i.e. the log of a number that is inversely proportional to the concentration of the immunoglobin, similar to the log of the dilution factor) is then plotted vs. log counts per minute (average of triplicates). The hypothesis that the fitted lines are parallel is tested using analysis of variance. It is found that the lines are parallel at a probability level of less than or equal to 0.01. Lines of all preparations are parallel and a common slope is determined. The x-intercepts are calculated from the common slope and the difference in intercepts used to determine the difference in potency. By this procedure, monoclonal antibody PE1-1 is some 435 times more potent than the Bureau of Biologics reference hepatitis B immune globin. Since the commercial hepatitis B immune globulin preparations were found to be two-fold (or less) more potent than the Bureau of Biologics reference preparation, PE1-1 is at least 200 times more potent than the commercial hepatitis B immune globulin preparations on a weight basis.

### EXAMPLE 4

### A. Binding Kinetics

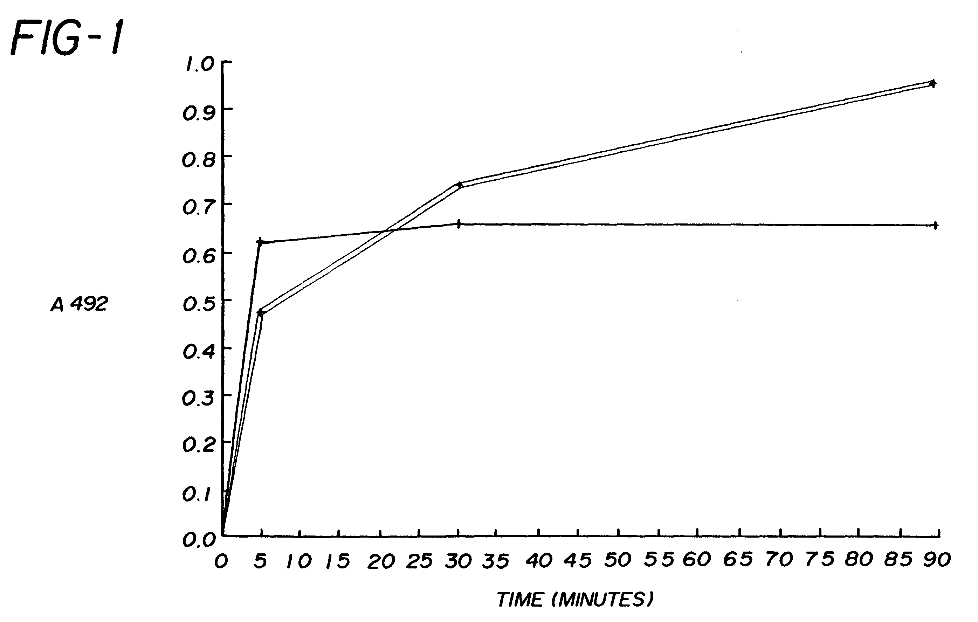

Direct binding enzyme linked immunoassays are used to compare the kinetics of binding to HBsAg of antibodies PE1-1 and ZM1-2. ELISA microtiter plates are coated with Heptavax® at 1 µg/ml. Wells are then incubated at 37°C with 2% fetal calf serum in phosphate buffered saline. Monoclonal antibody PE1-1 or ZM1-2 at 0.5 µg/ml in 2% fetal calf serum are incubated in the wells for various times. At the indicated times the antibody solution is removed and the well is rinsed three times with fresh 2% fetal calf serum. The well is then incubated with 2% fetal calf serum until the wells for the 90 minute time point contain 2% fetal calf serum. Thus, solution is then replaced with either peroxidase conjugated goat anti-lambda chain (PE1-1 wells) or goat anti-kappa chain (ZM1-2 wells). Quantitation of peroxidase conjugate bound to plastic is accomplished with the addition of O-phenylenediamine and H₂O₂. Results are presented in Fig. 1, where a single line is PE1-1 and a double line is ZM1-2.

As can be seen in Fig. 1, at a concentration at which PE1-1 is almost completely reacted in 5 minutes, the reaction of ZM1-2 with solid adsorbed HBsAg is not completed in 30 minutes and may continue to react for 90 minutes or more. Thus, PE1-1 binds significantly faster to antigen in this assay. Assuming this also occurs in vivo, PE1-1 is likely to be more efficient in neutralizing viral particles before they can infect the liver.

### B. Relative Position of Epitopes

The relative position of the epitopes of antibodies PE1-1, L03-3, and ZM1-2 are determined. A simultaneous sandwich immunoassay with a solid-adsorbed monoclonal antibody is used. The same antibody is radiolabelled and incubated in a microtiter well with the inhibitor and serum from a hepatitis E positive patient. Radiolabelled PE1-1 Fab fragment is used while radiolabelled L03-3 is intact IgG. Results are presented in Table 3, below.

**TABLE 3**

| Inhibition of Binding of Radiolabelled Monoclonal Antibody to HBsAg by Unlabelled Monoclonal Antibodies | | | |
|---|---|---|---|
| Solid-Absorbed maB | Iodinated maB | Inhibitor maB | IC₅₀ ng/ml |
| L03-3 | L03-3 | L03-3 | 10 |
| L03-3 | L03-3 | PE1-1 | >22,500 |
| PE1-1 | PE1-1 | PE1-1 | 8 |
| PE1-1 | PE1-1 | ZM1-2 | 76 |
| PE1-1 | PE1-1 | L03-3 | >22,500 |

Monoclonal antibody ZM1-2 is only approximately nine times less effective in inhibiting ¹²⁵I-PE1-1's binding to HBsAg than unlabelled PE1-1, whereas L03-3 is thousands of times less effective. Thus, the epitopes of ZM1-2 and PE1-1 are probably near each other on the HBsAg molecule while the L03-3 epitope is probably on a different part of the molecule. The reciprocal experiment, PE1-1 inhibition of radiolabelled L03-3, provides further evidence that PE1-1 and L03-3 bind to epitopes that are not overlapping.

The similarity of PE1-1 and 2M1-2 epitopes and their difference from the L03-3 is confirmed by immunoassay with reduced and alkylated HBsAg. L03-3 can bind to denatured antigen while both ZM1-2 and PE1-1 cannot so bind. It should be noted that PE1-1 and ZM1-2 have distinct epitopes since their reaction with different subtypes varies.

### C. Pharmokinetics of PE1-1 in Rhesus Monkeys

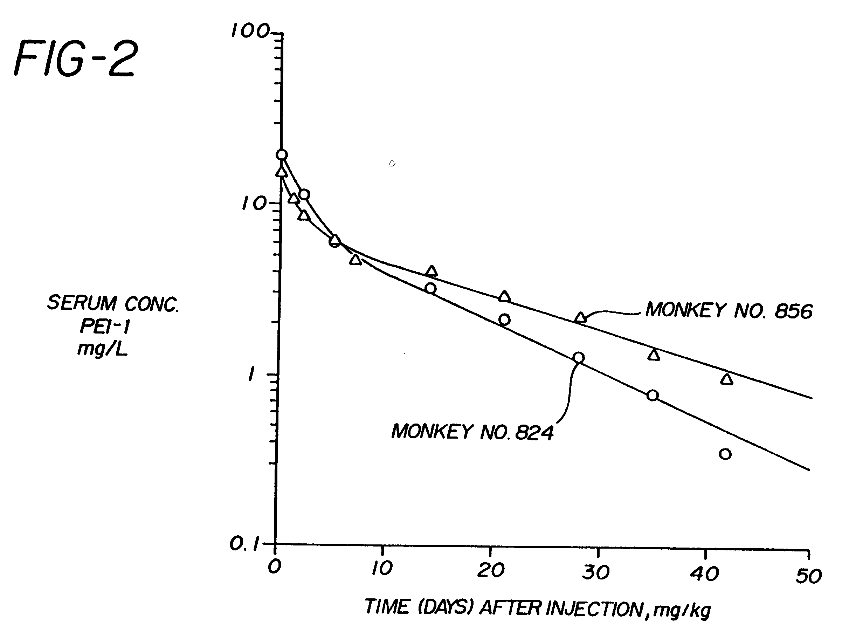

The pharmokinetics of PE1-1 is studied in two rhesus monkeys. Each animal receives a single intravenous bolus injection (0.5 mg/kg) of monoclonal antibody PE1-1. Serum levels of PE1-1 are determined at various times post-dose using an ELISA based sandwich immunoassay with Heptavax® coated on ELISA plates and rabbit anti-idiotypic antibodies to PE1-1. Results are shown in Figure 2.

Serum levels of PE1-1 in the two rhesus monkeys are characterized by a biphasic decline (t 1/2α = 1 and 1.4 days; t 1/2β = 11 and 16 days) with the shorter half-life possibly associated with the distribution phase of the monoclonal antibody. The volume of distribution at steady state (Vdss) is calculated to be 114-144% of the plasma volume, which suggests little distribution of PE1-1 to a tissue compartment in the antigen-free monkey.

### EXAMPLE 5

### CLINICAL TRIALS

### A. Compassionate use of PE1-1 in two patients with end-stage liver disease secondary to chronic active hepatitis B and hepatocellular carcinoma undergoing liver transplantation

PE1-1 was provided on a compassionate need basis to two patients with end-stage liver disease undergoing liver transplantation. Patient #1 was a 56 year old male with a 20 year history of chronic active hepatitis and a diagnosis of hepatocellular carcinoma. The second patient was a 10 year old male thought to have been infected with hepatitis B at birth. Patient #2 was initially evaluated for a large mass in the right lobe of the liver, which a biopsy confirmed was hepatocellular carcinoma.

Preoperative doses of PE1-1 were administered to these patients and significantly reduced their circulating HBsAg levels before the transplant procedure. Each patient also received two 20 mg doses of PE1-1 during transplantation. Postoperative dosing then began on the second day following surgery.

Patient #1 never became HBsAg negative, although his circulating HBsAg levels did diminish markedly from their pretreatment level. Patient #2 became HBsAg negative, first noted on post-transplant day 9. Patient #1 received additional doses of PE1-1 ranging from 5-40 mg at 2-20 day intervals. Patient #2 received either 5 or 10 mg doses on average of every 21-28 days.

No adverse events were reported for either of these patients during the period they received PE1-1. However, approximately four weeks after Patient #1 was discharged from the hospital, it was determined that he had metastatic malignancy. He expired on post-transplant day 139. No evidence of recurrent hepatitis was noted during his post-transplant course despite the presence of detectable circulating HBsAg. Although a hepatitis B virus DNA assay was negative preoperatively, a single positive value was detected 60 days post-transplant.

On post-transplant day 143, Patient #2 was first seen to be positive for HBsAg. The HBsAg level fluctuated for a short time before it then stabilized at a level signicantly below his pretreatment levels. Isolates of this patient's hepatitis B virus obtained before treatment with PE1-1 and at later times were analyzed for their binding ability to PE1-1. PE1-1 was found to be able to bind to the variant virus, but not as well as it had to the wild-type virus.

Genetic analysis of the two viral isolates indicated single nucleotide differences in a highly conserved region of the major viral surface protein. Such differences, when compared to the pre-treatment virus, could potentially encode for a single amino acid difference which would reduce the binding ability of PE1-1 to the hepatitis B viral binding particle.

### B. Use of PE1-1 in patients with chronic active hepatitis B undergoing liver transplantation (not complicated by hepatocellular carcinoma)

This study involved five patients who were HBsAg positive (but did not have hepatocellular carcinoma) and who underwent liver transplantation. Each patient was administered three daily preoperative doses of PE1-1, (10, 20 and 40 mg, respectively) over a three day period. The liver transplants were then performed from a minimum of two days to a maximum of 32 days following their preoperative dose of the study drug. An additional 40 mg dose of PE1-1 was administered during the operation. All five transplants were successfully completed.

The patients' HBsAg titers, liver enzymes, and other clinical parameters were closely monitored during their hospital stays. Follow-up evaluations and administration of PE1-1 by each patients' private physician continued on a regular basis (approximately every 1 to 3 weeks). Dosing and other parameters varied from patient to patient.

Two patients (#5 and #6) had similar results to Patient #2, above, in that a variant virus appeared after a period of negative HBsAg screening results. The sera of these patients remained active with PE1-1. Sequence analysis indicated the presence of single nucleotide differences between the variants in the patients' sera and wild type virus. Two variants were detected in each patient. Immunoassays and sequence analysis indicated that the variants in each patient were different and they also differed from the variants of Patient #2.

Patient #3 is a 39 year old Caucasian male who had end-stage liver disease secondary to a 16 year history of chronic hepatitis B. The three preoperative doses of PE1-1 that were administered to Patient #3 caused a substantial reduction in his HBsAg titer. On post-transplant days 2 and 3, he received 20 mg of PE1-1 and was first noted to be HBsAg negative on post-transplant day 2. For two months thereafter, Patient #3 received 10 mg PE1-1 on an average of every 1 to 7 days. Since then, he has received 7.5 or 10 mg doses of PE1-1 every 14 to 43 days. Histopathological evaluation of a liver biopsy performed in February, 1969 was negative for both HBsAg and HBcAg. Patient #3 remains HBsAg negative 582 days after transplant. In addition to receiving PE1-1, he has also received three consecutive monthly injections of Recombivax® in July, August and September, 1989.

Patient #4 was a 40 year old Arabic female who had end-stage liver disease secondary to a 10ö year history of chronic active Hepatitis B. The three preoperative doses of PE1-1 given to Patient #4 caused a substantial reduction in her HBsAg level. Patient #4 received 20 mg of PE1-1 on post-transplant days 1 and 2, and was found to be HBsAg negative on post-transplant day 6. For two months thereafter, she received 10 mg PE1-1 on average of every 3 to 8 days. Since then, she received 10 mg PE1-1 every 5 to 26 days. Approximately 1 year after her transplant, the patient developed hepatic artery thrombosis, but remained HBsAg negative, and was re-transplanted. Three days later, due to ischemia, a third transplant was performed. Twenty days following, a fourth transplant was performed due to infection. The patient expired 18 days after the fourth transplant (404 days after her initial transplant), secondary to liver failure and bacterial sepsis. Histopathological evaluation of a liver biopsy from her first transplanted liver showed that she was HBsAg negative.

Patient #5 is a 38 year old Caucasian male who had end-stage liver disease secondary to chronic active hepatitis B. The preoperative doses of PE1-1 administered to the patient substantially lowered his circulating HBsAg level. Patient #5 received 20 mg PE1-1 on post-transplant days 2 and 3, and was found to be HBsAg negative on post-transplant day 3. During the first two months post-transplant, he received 10 mg PE1-1 on average of every 3-7 days. Later, he received 10 mg PE1-1 every 9 to 26 days. The patient was noted to be HBsAg positive on post-transplant day 252, although his antigen level is substantially lower than his pre-transplant level. Histopathological evaluation of a liver biopsy performed in January 1990 is positive for HBsAg and HBcAg.

Patient #6 is a 38 year old Caucasian male who had end-stage liver disease secondary to chronic active hepatitis B and alcohol abuse. This patient acquired his initial infection via a blood transfusion. Prior to the transplant, he was positive for both HBsAg and HBeAg. Each preoperative dose of PE1-1 caused a decrease in the level of the patient's HBsAg titer. Patient #6 received 20 mg of PE1-1 on post-transplant days 1 and 2 and was noted to be HBsAg negative on post-transplant day 1. For two months thereafter, Patient #6 received 10 mg of PE1-1 on average of every 3-14 days. Subsequently he has received 10 mg PE1-1 every 7 to 63 days on an outpatient basis. The first HBsAg positive response was noted on post-transplant day 251 and occurred after his longest duration (63 days) between doses of PE1-1. Although at present Patient #6 is positive for HBsAg, his titer remains significantly lower than pre-transpiant levels.

Patient #7 is a 38 year old Caucasian female with a history of IV drug.abuse. This patient had end-stage liver disease secondary to chronic active hepatitis B. Prior to transplantation, the patient was positive for HBsAg and HBeAg. Each preoperative dose of PE1-1 caused a decrease in the patient's HBsAg titer. The first month post-transplant, Patient #7 received between 10 and 40 mg of PE1-1 on the average of every 1-7 days, and was noted to be HBsAg negative on post-transplant day 16. Subsequently, she received 10 mg PE1-1 every 15 to 29 days. Histopathological evaluation of a liver biopsy performed in July, 1989 was negative for HBsAg and HBcAg. Patient #7 remains HBsAg negative 464 days post-transplant.

### EXAMPLE 6

### REACTIVITY WITH VARIANT VIRUSUS

The reactivity of the monoclonal antibodies PE1-1, ZM1-2, and L03-3 with variant hepatitis B viruses isolated from patients described in Example 5 is investigated. Radioimmunoassays are performed by determining the radioactivity bound to a solid phase adsorbed-antibody. A solution of a monoclonal antibody at a concentration of 20µg/ml in phosphate-buffered saline containing 0.02% NaN₃ is incubated for at least 18 hours in U-bottom wells (Falcon MicroTest III Flexible Assay Plates). The solution is removed from the wells and the wells are then washed three times with distilled water. Fetal calf serum at a concentration of 2% in phosphate-buffered saline is added and incubated overnight at room temperature with solutions of serum HBsAg or controls and ¹²⁵I-radiolabelled antibody (approximately 4,000 cpm in 1% fetal calf serum). Wells are then washed with distilled water three times. Individual wells are excited and counted. Results are presented in Table 4, below.

**TABLE 4**

| Relative Reactivity of Serum-Derived Variant HBsAg with HBsAg-Specific Monoclonal Antibodies | | | |
|---|---|---|---|
| Sample* | L03-3:L03-3** | PE1-1:ZM1-2 | ZM1-2:ZM1-2 |
| Control | 1.000 | 1.000 | 1.000 |
| Patient #2 (234) | 0.013 | 0.070 | 0.233 |
| Patient #4 (251) | 0.007 | 0.024 | 0.010 |
| Patient #3 (264) | 0.043 | 0.173 | 0.179 |

| | | | |
|---|---|---|---|
| *Representative HBsAg-positive serum samples derived from patients after liver transplantation and treatment with an anti-HBsAg therapeutic monoclonal antibody PE1-1. Numbers in parentheses denote days after transplantation. | | | |
| **L03-3:L03-3 indicates a radioimmunoassay composed of both solid-adsorbed and radiolabelled human monoclonal antibody L03-3. PE1-1:ZM1-2 indicates a radioimmunoassay composed of human monoclonal antibody PE1-1 solid-adsorbed and human monoclonal antibody ZM1-2 radiolabelled. ZM1-2:Zm1-2 indicates a radioimmunoassay composed of both solid-adsorbed and radiolabelled human monoclonal antibody ZM1-2. Control KBsAg-positive serum reacted well with antibodies L03-3, PE1-1 and 2M1-2. | | | |

### EXAMPLE 7

### LARGE SCALE PRODUCTION OF ANTIBODIES

To initiate a production run with cells, one or more ampule(s) of frozen cells is removed from liquid nitrogen. After rapidly heating in a 37°C water bath until most of the ice has melted, the ampule is opened inside a vertical laminar flow hood. The contents of the ampule are mixed with a 1 ml volume of Dulbecco's MEM/Ham's F12(1:1) (DMEM/F12) to which ferric salts have been added to a final concentration of 50 µM of Feööö. After mixing, the tube is filled up to approximately 10 ml with the same medium and the cells are collected by centrifugation. The cell pellet is resuspended into 5 ml of the above mentioned medium with 20% fetal bovine serum and seeded into 1 well of a 6-well tissue culture plate. The cells are incubated in a 37°C incubator in a 5% CO₂-atmosphere. When the cells have established themselves in culture and start to multiply and have an approximate cell concentration of 10⁶/ml, the cells and the medium are moved into a tissue culture flask with a surface area of 80cm² and diluted to 40 ml using DMEM/F12 (without serum). When the cells have once again reached a concentration of 10⁶/ml, they, and the medium, are moved into a tissue culture flask with a surface area of 175cm² and further diluted to a volume of 100 ml using DMEM/F12. When the cells have once again reached optimal concentration, the cells and the medium are transferred into a roller bottle with a 850cm² surface area and diluted to a final volume of 500 ml. When this roller bottle has reached optimal cell concentration, it is split 1/3 into new roller bottles using the same medium as before. This splitting process of the roller bottles is continued until a sufficient number of bottles have been obtained in order to give a desired number of cells to seed into the Verax System 200 reactor.

### The Verax System 200

The Verax System 200 reactor is a closed cell culture system where cells are cultivated in stainless steel weighted microspheres (density 1.6g/mL) composed of cross-lined type I bovine collagen. The microspheres are loaded into a vertical transparent glass tube through which the culture medium (same as above) is pumped, entering at the bottom. The inlet to the tube is formed in such a fashion that the microspheres will establish a fluidized bed configuration when the medium is pumped through at a suitable velocity. During operation, fresh medium is constantly added and conditioned medium removed at a rate determined by the cell growth as monitored by glucose consumption. Temperature is maintained at 37°C; pH is maintained at 7.1 and oxygen/nitrogen ratio is also controlled.

After loading of the microspheres in 1% fetal bovine serum containing medium, the reactor is run for at least three days without cells to ascertain that the microsphere loading did not contaminate the System. During this time the reactor is fed with protein-free medium to reduce the priming dose of fetal bovine serum. If all systems are operating satisfactorily, the cells from the roller bottles are inoculated into the reactor.

### The Verax System 2000

This equipment uses the same type of microspheres as the system 200, and its controls and operations are essentially the same as for the smaller system. The System 2000 represents an approximately 15-fold scale-up compared to the System 200.

### Monitoring of Yield of Antibody from Full-Scale Culture

The conditioned medium is monitored, each time the harvest tank is emptied, for the level of human immunoglobulin in the supernatant using an ELISA-type assay. The results are confirmed using a Protein A HPLC method.

### Harvesting of Cell Culture Media and Production of Harvest Pool

The conditioned medium is continuously being removed from the Verax equipment into a refrigerated harvest tank. This medium is later unloaded (using the nitrogen pressure in the Verax system) into a mobile stainless steel tank for further processing.

### Cell Culture Media

The media routinely used is a 1:1 mixture of Dulbecco's MEM H21 and Ham's F12 (Mediatech). The medium is purchased as a powder sufficient for 50 liters of finished medium. Two such containers of each medium powder are added into a stainless steel tank containing approximately 190 liters of water. The powder is suspended with an impeller until all has been dissolved. Sodium bicarbonate is added as recommended by the manufacturer and the pH of the medium is set to 7.4. Sodium selenite is added to a final concentration of 17.3 µg/l and the volume is topped up to 2001 with water. The medium is also supplemented with ferric ions in the form of ferric nitrate/sodium citrate to a final concentration of 50 µM Fe+++. The medium is immediately added to the medium tank of the Verax System S200 through the built-in sterilization filter. No protein is added to the medium. No antibiotics of any type are ever used.

### PURIFICATION OF THE MONOCLONAL ANTIBODY

### Description of Methodology of Harvesting and Purification of End Product

The monoclonal antibody is produced in cell culture from a hybridoma cell line in the absence of serum. This means that we have a need to remove from the final product only components from the cellular material. As human monoclonal antibodies are not in themselves expected to be immunogenic, it becomes very important to remove all potentially immunogenic components.

The goal of the purification procedures is a final product that is more than 99.9% pure, using affinity chromatography. We depend heavily on the biological specificity of affinity chromatography. Each step of the purification process (summarized in Table 5) is discussed in more detail, supra.

**TABLE 5**

| Purification Summary | | |
|---|---|---|
| Step | Conditions | Materials |
| Cell Removal | Room Temp. | Polyvinylidene difluoride filters, 0.65/0.45 µm absolute. |
| | | |
| Concentration | | |
| Microfiltration | `4°C | Polysulfone filter nominal 30,000 daltons. Polyester (0.8µm) and cellulose acetate (0.2µm) absolute filters. |
| | | |
| Protein A chromatography | 4°C | Agarose coupled Staphyloccocus aureus Protein A. |
| | | |
| Concentration | 4°C | Cellulose triacetate filter, nominal 20,000 dalton. |
| | | |
| Gel chromatography | 4°C | Sephacryl S-300, Ringer's Lactated Solution |
| | | |
| Ion exchange | 4°C | Sephacryl S-300, Ringer's Lactated Solution |

### Cell Harvest and Removal of Particulate Materials from the Conditioned Medium

Even though most of the cells are retained by the microspheres, a sizable number of cells are present in the harvested supernatant. To avoid gross contamination of the medium by cell components the supernatant is filtered through a polyvinylidene difluoride 0.65µm Prostack® filter (Millipore), immediately after removal from the Verax harvest tank. This type of filter unit works in a tangential flow mode which allows filtration of large amount of particulate material without clogging the filter. The cleared medium is collected into a refrigerated stainless steel tank.

### Concentration of Conditioned Medium

The conditioned medium is concentrated using a nominal 30,000 dalton polysulfone spiral wound membrane supplied by Millipore. After concentration, the pH is set to 7.0 using 1M acetic acid. The material is sterile filtered through a Sartobran-PH 0.8/0.2µm (Sartorius) filter (the 0.8µm component is polyester, the 0.2µm component is cellulose acetate) before being stored at 4°C. The material is microfiltrated (0.22 µM Millipore) and filled into polypropylene vessels.

### Protein A Chromatography

The extremely powerful purification step utilizes the high affinity of the human IgG1 antibody to Staphylococcus aureus Protein A.

The Protein A is purchased already coupled covalently by an amide bond to agarose. After packing the gel in a column, the column with its contents and attached tubing is sanitized by treatment with 70% ethanol in water for 24 hours. The column is then equilibrated with PBS, pH 7.0.

Performing the affinity chromatography separation on the Protein A column involves the following sequential steps:
A) Loading. The concentrated conditioned medium is loaded on the column with a pump. The effluent from the column is collected and monitored for the presence of antibody by the human immunoglobulin ELISA. The column is loaded to such a degree that a measurable amount of antibody-containing fluid passes through the column. The overload fraction is separately recovered and recycled if it contains more than 20 mg/ml antibody.
B) Washing. To remove unbound materials the column is extensively washed with phosphate buffered saline, pH 7 with sodium chloride added to a final concentration of 0.5M. This wash is followed by a second washing step using a buffer of 0.02M sodium citrate, pH 5.6, containing 0.5M sodium chloride. This wash releases small amounts of the human antibody.
C) Elution. The bound monoclonal antibodies are eluted from the column using a buffer composed of 0.02M sodium citrate, pH 3.0, containing 0.5M sodium chloride. The eluted material is continuously diluted into a volume of 1M Tris-HCl, pH 8.0 to rapidly restore near-neutral conditions.

The Protein A purification is performed in a closed system utilizing a Waters 650 Protein Purification System.

### Concentration of Protein A Column Eluate

In order to make the following purification step more effective and convenient, the eluate from the Protein A column is concentrated to at least 5 mg/ml antibody. The concentrate is sterile filtered through a 0.2 µm filter and the sterile concentrate is stored at 4°C until sufficient materials have been collected for the next purification step.

### Size Separation by Gel Chromatography on Sephacryl S-300 High Resolution

The antibody preparation is run on a Sephacryl S300 High Resolution (Pharmacia) gel, packed in a Pharmacia BP113/120 column with a bed volume of approximately 10 liters. The column is packed in Lactated Ringer's Irrigation USP (Travenol Laboratories). The elution of the column is monitored by a Waters 650 Protein Purification System.

The purpose of this step is not principally additional purification, but buffer change. After the elution of the Protein A column the antibodies are in a complex, hypertonic buffer composed of sodium citrate, sodium chloride and Tris-HCl. This buffer mixture can not be used directly as a vehicle for an intravenous injection. The buffer after this step is suitable both for intravenous injection and for long term refrigerated storage.

### Removal of Host Cell DNA by Passage over an Ion Exchange Column

Even after the Protein A chromatography, which removes the bulk of DNA present in the concentrated supernatant, and the Sephacryl S-300HR which removes DNA molecules that are either significantly larger or significantly smaller than the monoclonal antibody product, there is a small; but detectable, presence of DNA in the antibody preparation. We have selected to remove this contaminant by an ion exchange step on a strong anion exchanger, Q Sepharose (Pharmacia Inc.). At the pH of Lactated Ringer's solution, antibody proteins have a positive charge, and are repelled by the anion exchanger. Nucleic acids, however, have a negative charge at this pH, and will bind to the column.

The column was packed according to the manufacturer's suggestions. After decanting the 20% ethanol solution the gel is delivered in, 100 ml of gel was suspended in 200 ml of Lactated Ringer's solution. The slurry is poured into a Pharmacia K50/30 column, and when the gel has packed itself to a constant volume, it is sanitized with 1 column volume of 0.5N sodium hydroxide, followed by 3 column volumes of Dulbecco's PBS, followed by 5 column volumes of Lactated Ringer's solution. Immediately prior to use the column was washed with an additional 5 column volumes of Lactated Ringer's solution. The sample is then passed through the column and the pass-through is collected in a sterile container.

### EXAMPLE 8

### MOLECULAR ANALYSES OF PE1-1, ZM1-1, ZM1-2 AND MD3-4

The heavy variable (V_{H}) chain of antibodies PE1-1, ZM1-1, ZM1-2 and MD3-4 are isolated and sequenced. Total RNA is extracted from 10⁷ hybridoma cells of each cell line using procedures described in Sanz, et al. 1989 J. Immunol. 142:883, which is hereby incorporated by reference. Single stranded DNA is synthesized using AMV-reverse transcriptase as the enzyme and oligo-dT as the primer. The quantity of the synthesized ss-cDNA is assessed by measuring the incorporation of ³²p-dCTP.

Polymerase chain reactions (PCR) are performed essentially as recommended by the manufacturer (Perkin Elmer Cetus, Norwalk, Connecticut). One microgram of DNA is added to a 200 µm solution of each of dATP, dCTP, dGTP and dTTP, with 100 p moles each of primer and 5 units of Taq DNA polymerase. PCR cycles are as follows: denaturation at 98°C for 3 minutes, annealing at 55°C for 2 minutes, and extension at 72°C for three minutes, controlled in a DNA thermal cycler (Perkin Elmer Cetus).

Amplified DNA is size selected on a 1.0% low melting agarose gel, ligated into the EcoRV site of a BLUESCRIPT phagemid vector, and transformed into CaCl₂ competent BSJ72 bacteria. Single stranded DNA for sequencing is isolated from each positive clone after superinfection with M13K07 as described by Sanz, et al., supra. Sequencing is accomplished via the dideoxy chain termination method as described by Sanger, et al. 1980 J. Mol. Biol. 143:161, except a modified T7 DNA polymerase (Sequenase) is used as described by Tabor, et al. 1987. PNAS (USA) 84:4767. Results are given in Tables 8-1, 8-2, 8-3 and 8-4.

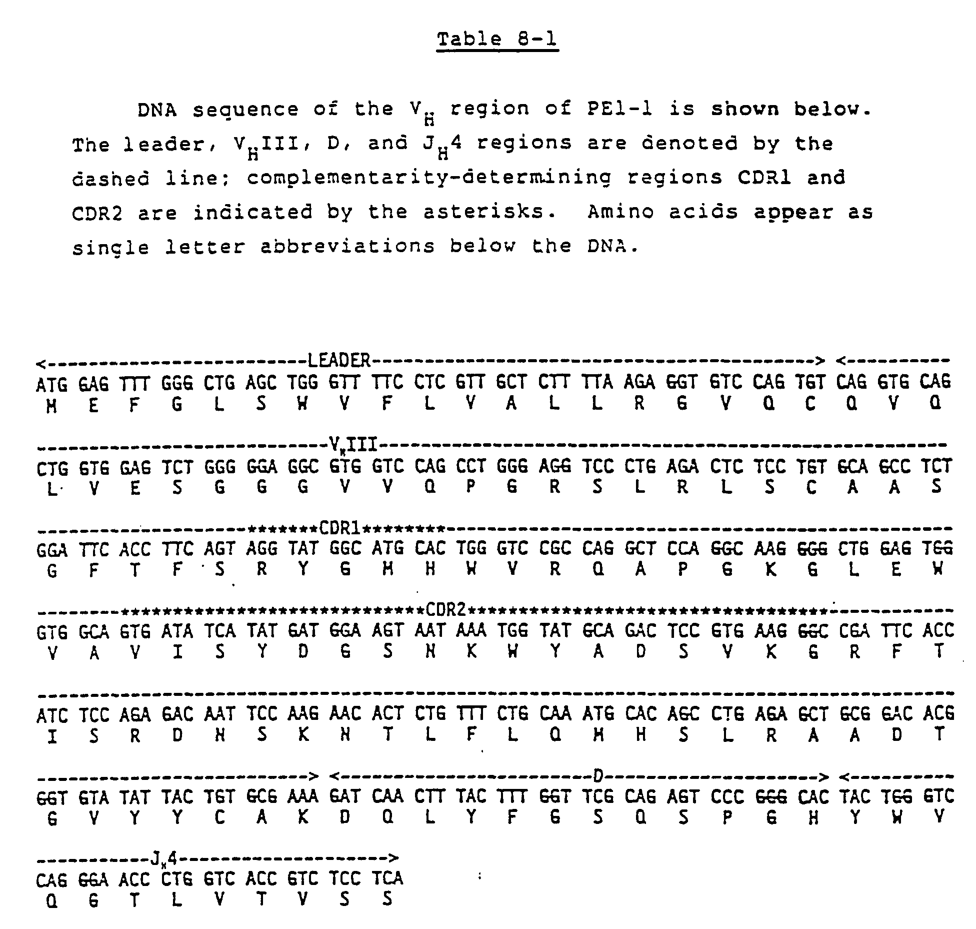

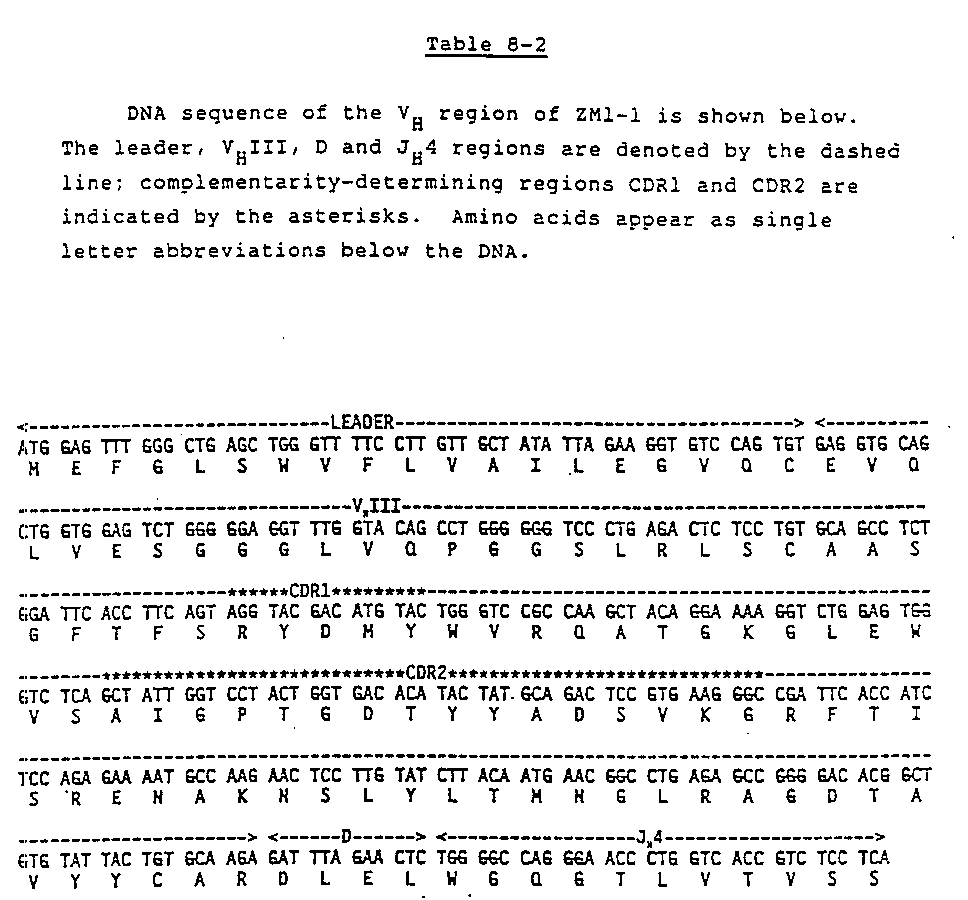

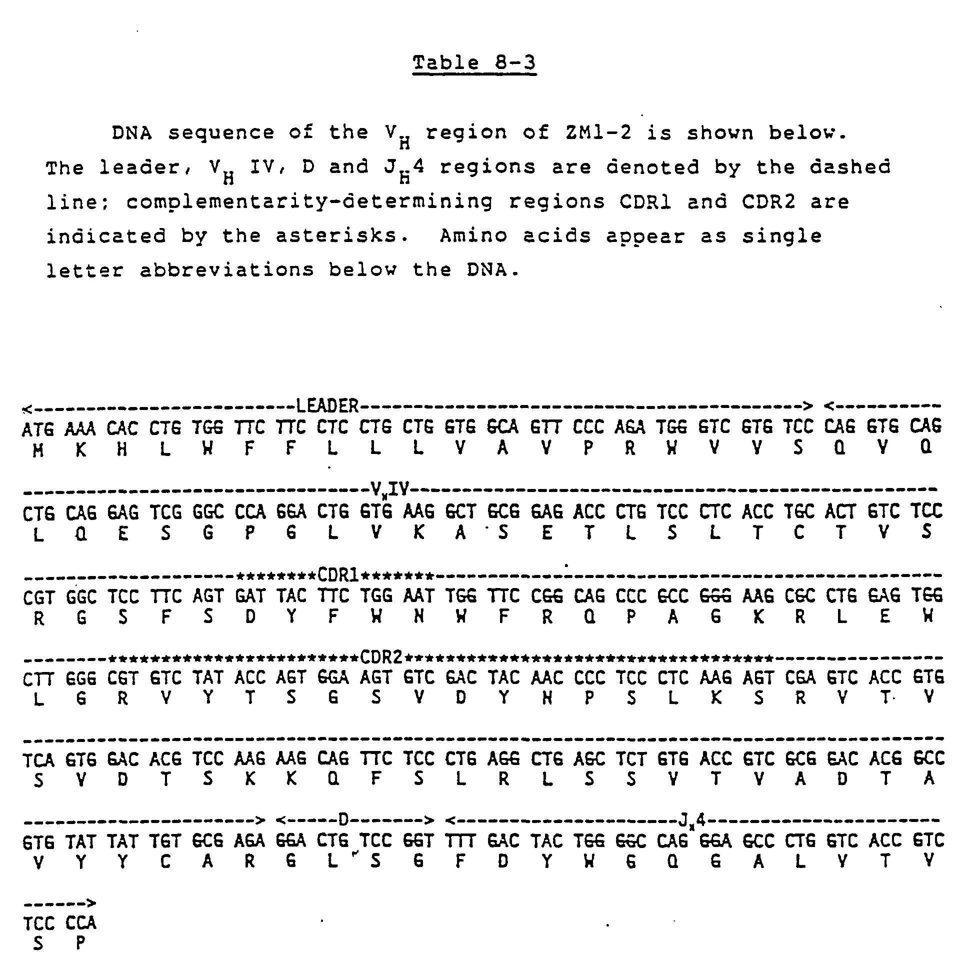

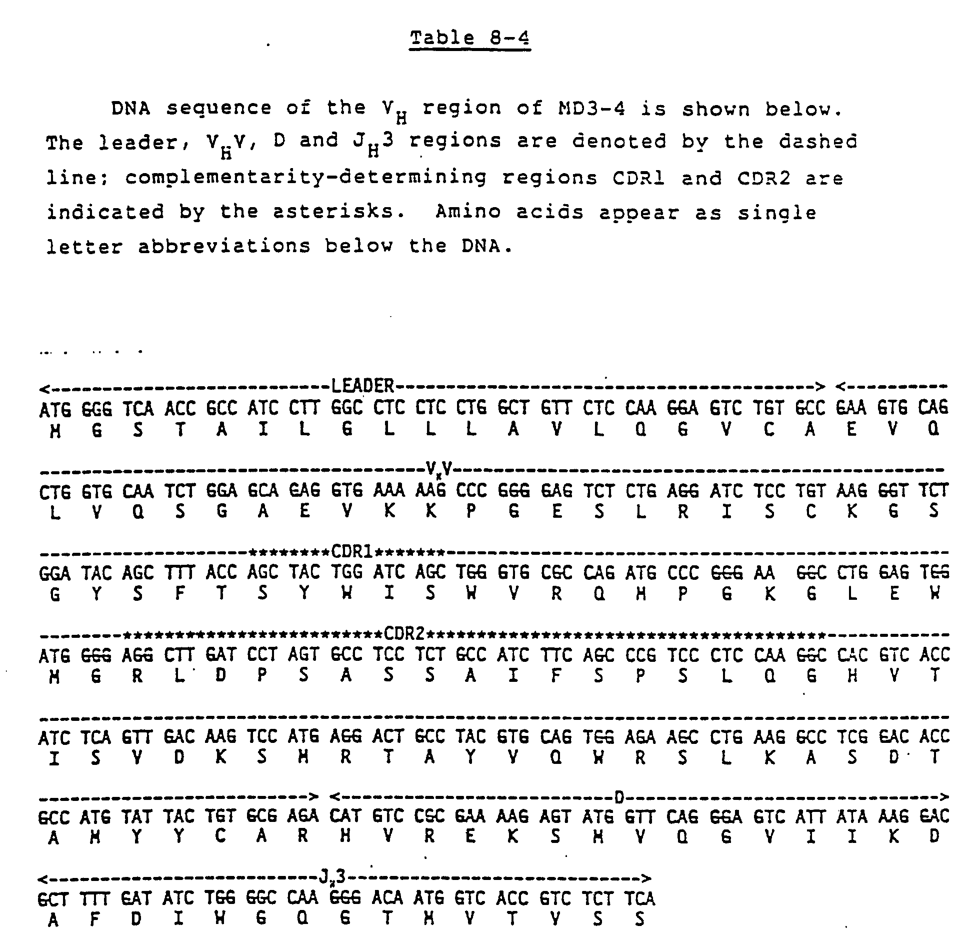

### EXAMPLE 9

Following the procedures of Example 8, the light variable (V_{L}) chain of antibodies PE1-1, ZM1-1, ZM1-2 and MD3-4 are isolated and sequenced. Results are given in Tables 9-1, 9-2, 9-3 and 9-4.

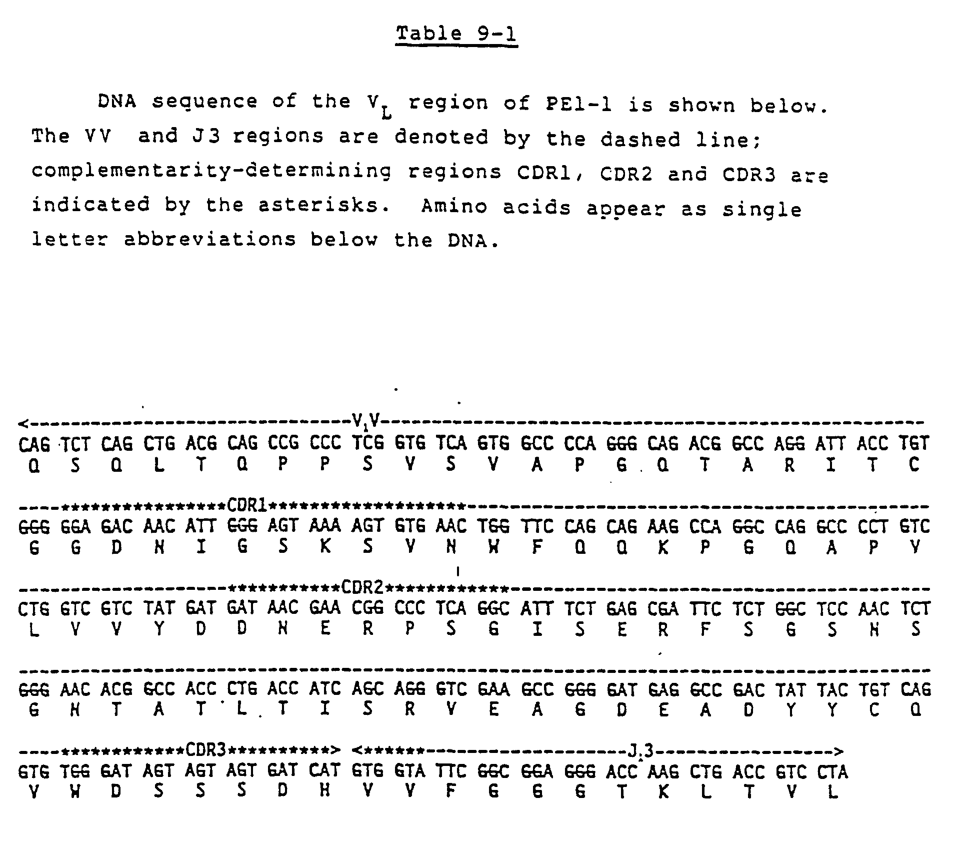

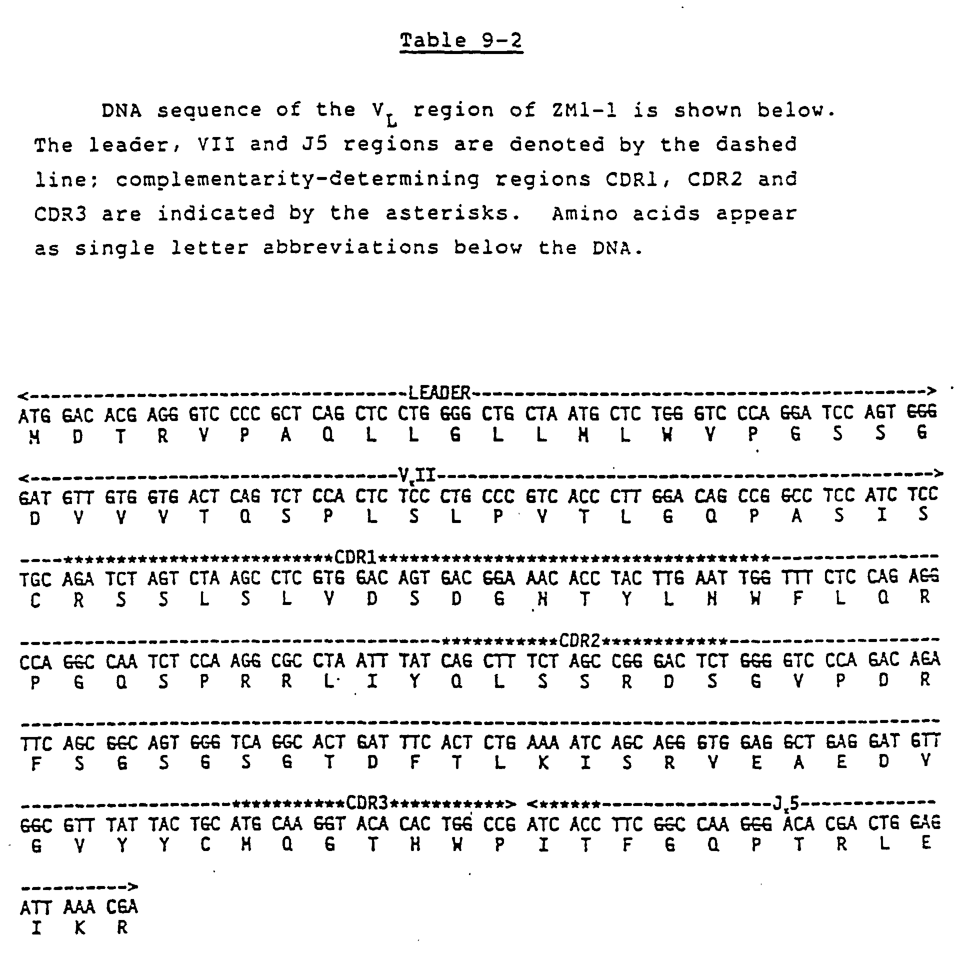

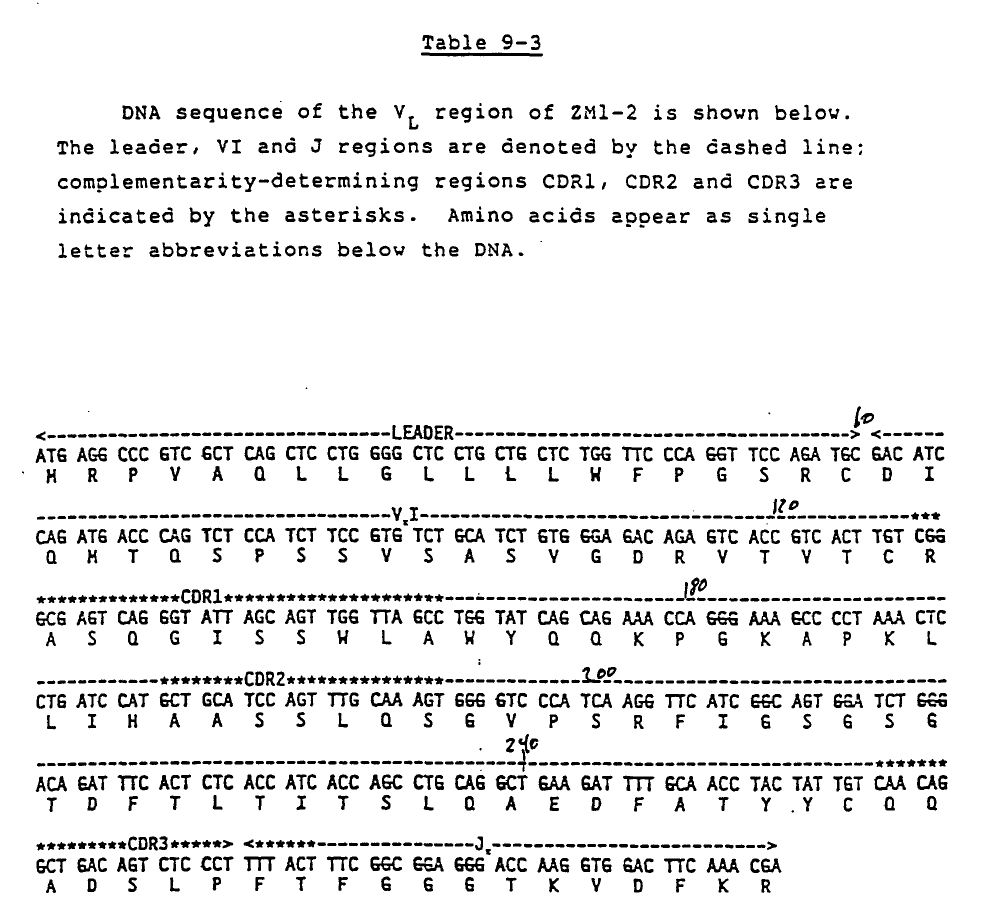

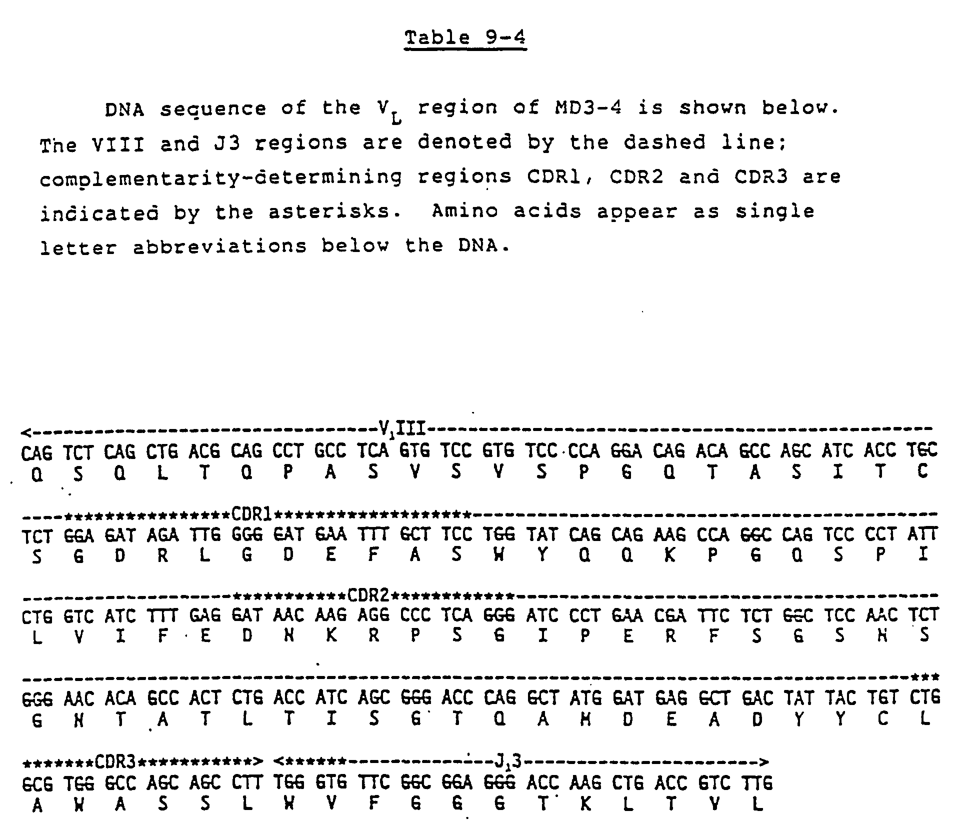

## Claims

1. A human monoclonal antibody or Fab fragment thereof, which neutralises hepatitis B virus, and has a V_{H} region with the amino acid sequence shown in Table 8-1 and which has a V_{L} region with the amino acid sequence shown in Table 9-1.

2. An antibody as claimed in claim 1 wherein the antibody is of IgG₁ class and comprises a lambda light chain.

3. A human monoclonal antibody that neutralises hepatitis B virus and which has a V_{H} region which has at least 80% sequence identity to that shown in Table 8-1 and which has a V_{L} region which has at least 80% sequence identity to that shown in Table 9-1, wherein the monoclonal antibody inhibits binding of an antibody of claim 1 to hepatitis B surface antigen.

4. An antibody as claimed in claim 3 that is a Fab fragment.

5. A hybridoma cell line comprising a xenogeneic immortalising cell fused to a human cell capable of producing an antibody as claimed in any of claims 1 to 3.

6. A cell line as claimed in claim 5, wherein the xenogeneic immortalising cell comprises a hybridoma fused from a parent immortalising cell and a human partner cell.

7. A cell line as claimed in claim 6, wherein the parent immortalising cell is a murine myeloma or hybridoma.

8. A cell line as claimed in any of claims 5 to 7 which is capable of a producing human monoclonal antibody as claimed in claim 1.

9. A method of making the hybridoma cell line as claimed in any of claims 5 to 8, comprising making a xenogeneic immortalising cell line drug resistant, fusing the resulting drug resistant immortalising cell to a cell capable of producing human antibody, and selecting the desired hybrid.

10. A cell line which produces an antibody as claimed in claim 3.

11. A method of producing an antibody as claimed in any of claims 1 to 3, comprising obtaining said antibody from a cultured hybridoma cell line as claimed in any of claims 5 to 7 or claim 10.

12. A method as claimed in claim 11 wherein the method further comprises forming the antibody into a dosage form for administration to a patient.

13. Isolated DNA, optionally as a component of a vector, comprising a DNA sequence which hybridises under stringent conditions to a strand of DNA selected from:
a) a strand of Table 8-1; and
b) a strand of Table 9-1;
wherein the DNA encodes an antibody chain, which as a component of an antibody inhibits binding of an antibody as claimed in claim 1 to hepatitis B surface antigen.

14. Use of DNA as claimed in claim 13 as a probe or for use in cloning antibodies.

15. A cell line having DNA comprising the DNA of claim 13.

16. A pharmaceutical composition comprising an antibody or Fab fragment thereof, as claimed in any of claims 1 to 4.

17. An antibody or Fab fragment thereof as claimed in any of claims 1 to 4, which antibody or Fab fragment is for use as a pharmaceutical.

18. Use of an antibody or Fab fragment thereof as claimed in any of claims 1 to 4 for the manufacture of a medicament for combating hepatitis B virus and/or reducing the level of circulating hepatitis B surface antigen.

19. Use of an antibody or Fab fragment thereof as claimed in any of claims 1 to 4, in an in vitro diagnostic assay.

20. A use as claimed in claim 18 for treatment of a liver transplant patient.

21. A use as claimed in claim 18 for treatment of a patient with chronic active hepatitis.

## Patentansprüche

1. Menschlicher monoklonaler Antikörper oder Fab-Fragment davon, der Hepatitis B-Virus neutralisiert und eine V_{H}-Region mit der in Tabelle 8-1 gezeigten Aminosäuresequenz und eine V_{L}-Region mit der in Tabelle 9-1 gezeigten Aminosäuresequenz besitzt.

2. Antikörper gemäß Anspruch 1, wobei der Antikörper der IgG₁-Klasse angehört und eine lambda-leichte Kette umfasst.

3. Menschlicher monoklonaler Antikörper, der Heptatis B-Virus neutralisiert und eine V_{H}-Region mit wenigstens 80% Sequenzidentität zu der in Tabelle 8-1 gezeigten und eine V_{L}-Region mit wenigstens 80% Sequenzidentität zu der in Tabelle 9-1 gezeigten besitzt, wobei der monoklonale Antikörper die Bindung eines Antikörpers gemäß Anspruch 1 an Oberflächen-Antigen von Hepatitis B hemmt.

4. Antikörper gemäß Anspruch 3, der ein Fab-Fragment ist.

5. Hybridom-Zelllinie, umfassend eine xenogenetische immortalisierende Zelle in Fusion mit einer menschlichen Zelle, die einen Antikörper gemäß einem der Ansprüche 1 bis 3 produzieren kann.

6. Zelllinie gemäß Anspruch 5, wobei die xenogenetische immortalisierende Zelle ein Hybridom umfasst, das ausgehend von einer parentalen immortalisierenden Zelle und einer menschlichen Partnerzelle fusioniert wurde.

7. Zelllinie gemäß Anspruch 6, wobei die parentale immortalisierenden Zelle ein Mäuse-Myelom oder -Hybridom ist.

8. Zelllinie gemäß einem der Ansprüche 5 bis 7, fähig zur Produktion von menschlichem monoklonalen Antikörper gemäß Anspruch 1.

9. Verfahren zur Herstellung der Hybridom-Zelllinie gemäß einem der Ansprüche 5 bis 8, umfassend die Erzeugung einer Resistenz gegenüber einem Gift bei einer xenogenetischen immortalisierenden Zelllinie, Fusion der resultierenden Gift-resistenten immortalisierenden Zelle mit einer Zelle, die menschlichen Antikörper produzieren kann und Selektion des gewünschten Hybrids.

10. Zelllinie, die einen Antikörper gemäß Anspruch 3 produziert.

11. Verfahren zur Herstellung eines Antikörpers gemäß einem der Ansprüche 1 bis 3, umfassend die Gewinnung des Antikörpers von einer kultivierten Hybridom-Zelllinie gemäß einem der Ansprüche 5 bis 7 oder Anspruch 10.

12. Verfahren gemäß Anspruch 11, wobei das Verfahren ferner das Einverleiben des Antikörpers in eine Dosierungsform für eine Verabreichung an einen Patienten umfasst.

13. Isolierte DNA, gegebenenfalls als Bestandteil eines Vektors, umfassend eine DNA-Sequenz, die unter stringenten Bedingungen mit einem DNA-Strang hybridisiert, ausgewählt aus:
a) einem Strang der Tabelle 8-1; und
b) einem Strang der Tabelle 9-1;
wobei die DNA eine Antikörperkette kodiert, die als Bestandteil eines Antikörpers die Bindung eines Antikörpers gemäß Anspruch 1 an Oberflächen-Antigen von Hepatitis B hemmt.

14. Verwendung von DNA gemäß Anspruch 13 als Sonde oder für die Klonierung von Antikörpern.

15. Zelllinie mit DNA, die die DNA gemäß Anspruch 13 umfasst.

16. Pharmazeutische Zusammensetzung, umfassend einen Antikörper oder ein Fab-Fragment davon gemäß einem der Ansprüche 1 bis 4.

17. Antikörper oder Fab-Fragment davon gemäß einem der Ansprüche 1 bis 4, wobei der Antikörper oder das Fab-Fragment als Arzneimittel Verwendung findet.

18. Verwendung eines Antikörpers oder Fab-Fragments davon gemäß einem der Ansprüche 1 bis 4 für die Herstellung eines Arzneimittels für die Bekämpfung von Hepatitis B-Virus und/oder die Verminderung der Menge an zirkulierendem Oberflächen-Antigen von Hepatitis B.

19. Verwendung eines Antikörpers oder Fab-Fragments davon gemäß einem der Ansprüche 1 bis 4 in einem *in vitro*-diagnostischen Test.

20. Verwendung gemäß Anspruch 18 für die Behandlung eines Patienten mit einem Lebertransplantat.

21. Verwendung gemäß Anspruch 18 für die Behandlung eines Patienten mit chronischer aktiver Hepatitis.

## Revendications

1. Anticorps monoclonal humain ou fragment Fab de celui-ci, qui neutralise le virus de l'hépatite B et possède une région V_{H} contenant la séquence d'acides aminés présentée dans le tableau 8-1 ainsi qu'une région V_{L} contenant la séquence d'acides aminés présentée dans le tableau 9-1.

2. Anticorps selon la revendication 1, lequel anticorps appartient à la classe des IgG₁ et comprend une chaîne légère lambda.

3. Anticorps monoclonal. humain qui neutralise le virus de l'hépatite B et possède une région V_{H} dont la séquence présente une identité d'au moins 80% avec celle qui est présentée au tableau 8-1 et une région V_{L} dont la séquence présente une identité d'au moins 80% avec celle qui est présentée au tableau 9-1, lequel anticorps monoclonal inhibe la liaison d'un anticorps selon la revendication 1 à un antigène de surface de l'hépatite B.

4. Anticorps selon la revendication 3 qui est un fragment Fab.

5. Lignée de cellules d'hybridomes comprenant une cellule immortalisante xénogénique fusionnée avec une cellule humaine capable de produire un anticorps selon l'une quelconque des revendications 1 à 3.

6. Lignée cellulaire selon la revendication 5, dans laquelle la cellule immortalisante xénogénique comprend un hybridome fusionné à partir d'une cellule immortalisante parentale et d'une cellule partenaire humaine.

7. Lignée cellulaire selon la revendication 6, dans laquelle la cellule parentale immortalisante est un myélome ou un hybridome murin.

8. Lignée cellulaire selon l'une quelconque des revendications 5 à 7 qui est capable de produire un anticorps monoclonal humain selon la revendication 1.

9. Méthode de préparation de la lignée de cellules d'hybridome selon l'une quelconque des revendications 5 à 8, comprenant la préparation d'une lignée de cellules immortalisantes xénogéniques résistantes aux médicaments, la fusion de la cellule immortalisante résistante aux médicaments résultante à une cellule capable de produire un anticorps humain et la sélection de l'hybride désiré.

10. Lignée cellulaire qui produit un anticorps selon la revendication 3.

11. Procédé de production d'un anticorps selon l'une quelconque des revendications 1 à 3 comprenant la récupération dudit anticorps à partir d'une lignée cellulaire d'hybridome en culture selon l'une quelconque des revendications 5 à 7 ou selon la revendication 10.

12. Procédé selon la revendication 11, lequel procédé comprenant en outre la formation de l'anticorps sous une forme destinée à être administrée à un patient.

13. ADN isolé, éventuellement sous la forme d'un composant d'un vecteur, comprenant une séquence d'ADN qui s'hybride dans des conditions stringentes à un brin d'ADN choisi parmi :
a) un brin du tableau 8-1 ; et
b) un brin du tableau 9-1 ;
dans lequel l'ADN code pour une chaîne d'anticorps, qui, en tant que composant d'un anticorps, inhibe la liaison d'un anticorps selon la revendication 1 à un antigène de surface de l'hépatite B.

14. Utilisation de l'ADN selon la revendication 13 en tant que sonde ou pour cloner des anticorps.

15. Lignée cellulaire possédant l'ADN comprenant l'ADN selon la revendication 13.

16. Composition pharmaceutique comprenant un anticorps ou un fragment Fab de celui-ci, selon l'une quelconque des revendications 1 à 4.

17. Anticorps ou un fragment Fab de celui-ci, selon l'une quelconque des revendications 1 à 4, lequel anticorps ou fragment Fab étant destiné à être utilisé en tant que produit pharmaceutique.

18. Utilisation d'un anticorps ou d'un fragment Fab de celui-ci, selon l'une quelconque des revendications 1 à 4, pour la fabrication d'un médicament destiné à lutter contre le virus de l'hépatite B et/ou à réduire le taux d'antigène de surface de l'hépatite B circulant.

19. Utilisation d'un anticorps ou d'un fragment Fab de celui-ci selon l'une quelconque des revendications 1 à 4, dans un test diagnostique in vitro.

20. Utilisation selon la revendication 18 destinée au traitement d'un patient transplanté hépatique.

21. Utilisation selon la revendication 18 destinée au traitement d'un patient atteint d'une hépatite active chronique.
